# EUROPEAN PATENT APPLICATION

(11) **EP 1 973 075 A1**
(43) Date of publication of application: **24.09.2008**
(21) Application number: 07425158.8
(22) Date of filing: 20.03.2007
(51) Int. Cl.: G06T 7/00, G06T 15/00, A61B 6/00

(54) **Method for the reconstruction of a panoramic image of an object, and a computed tomography scanner implementing said method**

(71) Applicant: CEFLA SOCIETA' COOPERATIVA, 40026 Imola (BO) (IT)
(72) Inventor: Pasini, Alessandro, 47023 Cesena (IT); Bianconi, Davide, 40023 Castel Guelfo di Bologna (IT); Nanni, Eros, 40023 Castel Guelfo di Bologna (IT)
(74) Representative: Jorio, Paolo

(57) **Abstract**

In a computed tomography scanner, the panoramic image of an object to be analysed is reconstructed by: acquiring volumetric tomographic data of the object; extracting, from the volumetric tomographic data, tomographic data corresponding to at least three sections (17a-17c) of the object identified by respective mutually parallel planes; determining, on each section (17a-17c) extracted, a respective trajectory (20a-20c) that a profile of the object follows in an area corresponding to said section (17a-17c); determining a first surface (S) transverse to said planes such as to comprise the trajectories (20a-20c); and generating the panoramic image on the basis of a part of the volumetric tomographic data identified as a function of said surface (S).

## Description

The present invention relates to a method for the reconstruction of a panoramic image of an object and to a computed tomography scanner functioning according to said method.

In particular, the present invention finds advantageous, though non-exclusive, application in the computed tomography scanners used in the sector of dentistry, to which the ensuing description will make explicit reference without this implying any loss in generality.

In the dentistry sector, computed tomography scanners are used, in the present state of the art, of the type comprising an X-ray source-detector assembly designed to rotate about a support, which supports the head of a patient, for acquiring volumetric tomographic data of one or both of the dental arches of the patient, and a calculation unit, for example, a personal computer, connected to the source-detector assembly for receiving from the detector radiographic data and processing said data so as to reconstruct tomographic images of the dental arches. In particular, it is known to reconstruct, starting from the volumetric tomographic data acquired, panoramic images of the dental arches. Said panoramic images are reconstructed by selecting the tomographic data on a U-shaped or V-shaped curved surface substantially orthogonal to an axial plane of the patient ideally traced on the inside or on the outside of the arches.

Even though the panoramic image created in the way just described above is free from defects of magnification typical of orthopantomographs, it still presents imprecisions due to the fact of not taking into account the different inclinations of the teeth. On the sagittal plane, for example, the axis of the teeth of the upper arch is hardly ever perpendicular to the table of the hard palate, and the axis of the teeth of the lower arch is hardly ever perpendicular to the lower edge of the jaw. In addition, the teeth present morphologies and inclinations that are different from one another, and said characteristics vary from patient to patient. It follows that the panoramic image obtained in the way described above frequently does not represent all the teeth with their actual dimensions.

The aim of the present invention is to provide a method for the reconstruction of a panoramic image of the dental arches of a patient, a computer program product, and a computed tomography scanner implementing said method that are free from the drawbacks described above and, at the same time, are easy and inexpensive to produce.

Provided according to the present invention are a method for the reconstruction of a panoramic image of an object, a computer program product, and a computed tomography scanner and its uses in accordance with the annexed claims.

The present invention will now be described with reference to the annexed plates of drawings, which illustrate a nonlimiting example of embodiment thereof and in which:
- Figure 1 illustrates a computed tomography scanner of the type used in dentistry;
- Figure 2 shows an image of an axial section of a pair of dental arches obtained via the computed tomography scanner of Figure 1;
- Figure 3 shows, according to a perspective view, a combination of images of an axial section, of a sagittal section, and of a coronal section of the same dental arches of Figure 2 obtained via the computed tomography scanner of Figure 1; and
- Figures 4, 5 and 6 are schematic illustrations of the axial section of the image of Figure 2 and the combination of sections corresponding to the images shown in Figure 3, said sections being processed according to the method for the reconstruction of a panoramic image according to the present invention.

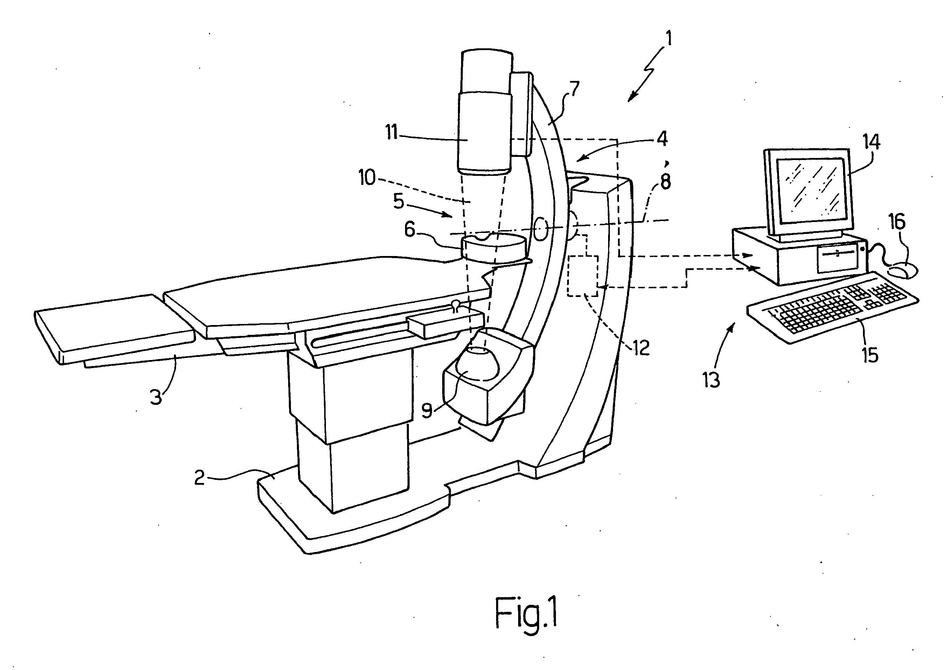

In Figure 1, designated, as a whole, by 1 is a computed tomography scanner of the type used in dentistry, comprising a frame 2, a couch 3 for supporting a patient (not illustrated) lying down, and an X-ray source-detector assembly 4 designed to rotate about an area of analysis 5 set in a region corresponding to a headrest 6 of the couch 3 for acquiring volumetric tomographic data of a part of the head of the patient, for example a maxillofacial complex such as the dental arches, the mandibular bone, or else the maxillary bone of the patient, which hereinafter will be referred to as "object" for reasons of simplicity. The position of the couch 3 is adjustable with respect to the frame 2 so as to enable correct positioning of the head of the patient in the area of analysis 5.

The source-detector assembly 4 comprises: an arm 7, mounted on the frame 2 and motor-driven so as to rotate about a substantially horizontal axis 8 traversing the region of analysis 5; an X-ray emitter 9, mounted on a first end of the arm 7 and facing in the direction of the axis 8 for emitting a conical beam 10 of X rays towards the area of analysis 5; and an X-ray detector 11, mounted on the opposite end of the arm 7 and facing in the direction of the axis 8 for receiving the beam 10 after it has traversed the area of analysis 5 and, hence, the object to be analysed.

The tomography scanner 1 further comprises: a control unit 12, connected to the source-detector assembly 4 for controlling emission and reception of the beam 10 in a way synchronous with rotation of the arm 7; and a processing unit 13, connected to the detector 11 for receiving, storing, and processing the volumetric tomographic data so as to reconstruct images of the object and to the control unit 12 for activating the source-detector assembly 4 on the basis of commands imparted by an operator or of instructions with which the processing unit 13 itself is programmed. The processing unit 13 is constituted, for example, by a personal computer provided with a monitor 14 for displaying the reconstructed images, and a keyboard 15 and a mouse 16 for acquiring data supplied and/or commands imparted by the operator.

Loaded into the processing unit 13 is a program for image reconstruction, which implements a method for the reconstruction of a panoramic image of an object in accordance with the present invention. Hereinafter, said method will be described with particular reference to reconstruction of a panoramic image of the dental arches of a patient.

The method for reconstruction of a panoramic image envisages, first of all, acquiring, via the source-detector assembly 4, volumetric tomographic data of the object to be analysed, i.e., the dental arches. Said acquisition is made according to known techniques, for example, by performing a complete rotation, about the axis 8, of the source-detector assembly 4 in order to receive, via the detector 11, projections of sections of the object at different angles of rotation. Starting from the projections of sections, the object is typically reconstructed according to planar slices that are identified by respective planes parallel to an axial plane, which is defined as a plane orthogonal to the axis 8, and that hereinafter will be referred to as "axial sections" of the object. The tomographic data of each axial section consist in values of absorption of the radiation (X rays) used associated to the volume elements, or "voxels", intercepted by the plane that identifies the axial section. The volumetric tomographic data acquired are then stored, in the form of digitized axial sections, in an internal memory of the processing unit 13.

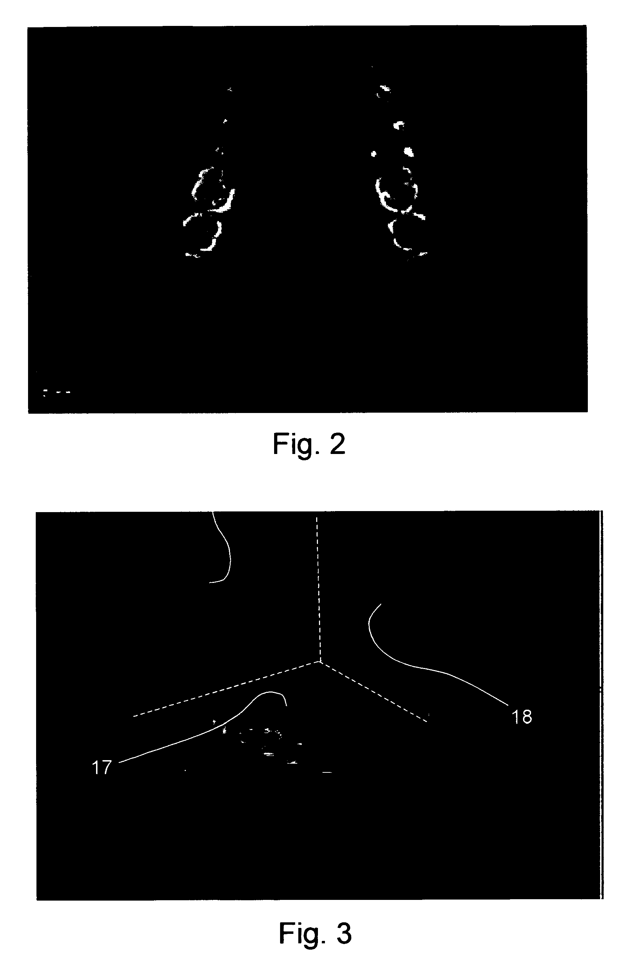

Figure 2 shows an image of an axial section of a dental arch acquired, via the tomography scanner 1, in an area corresponding to the patient's bite.

Figure 3 shows a combination, according to a perspective view, of the image of the axial section of Figure 2, said section being designated by 17, with the images of a sagittal section 18 and of a coronal section 19 corresponding to one and the same maxillofacial complex. The sections 17, 18 and 19 are perpendicular to one another. The images of the sagittal section 18 and coronal section 19 are reconstructed by processing the volumetric tomographic data obtained from the two-dimensional tomographic data of a plurality of axial sections, of the same type as the axial section 17, acquired by the tomography scanner 1.

According to the present invention, the method envisages extracting, from the volumetric tomographic data acquired, tomographic data corresponding to at least three axial sections 17; namely, at least three axial sections 17 are selected from amongst the plurality of axial sections acquired for the dental arches.

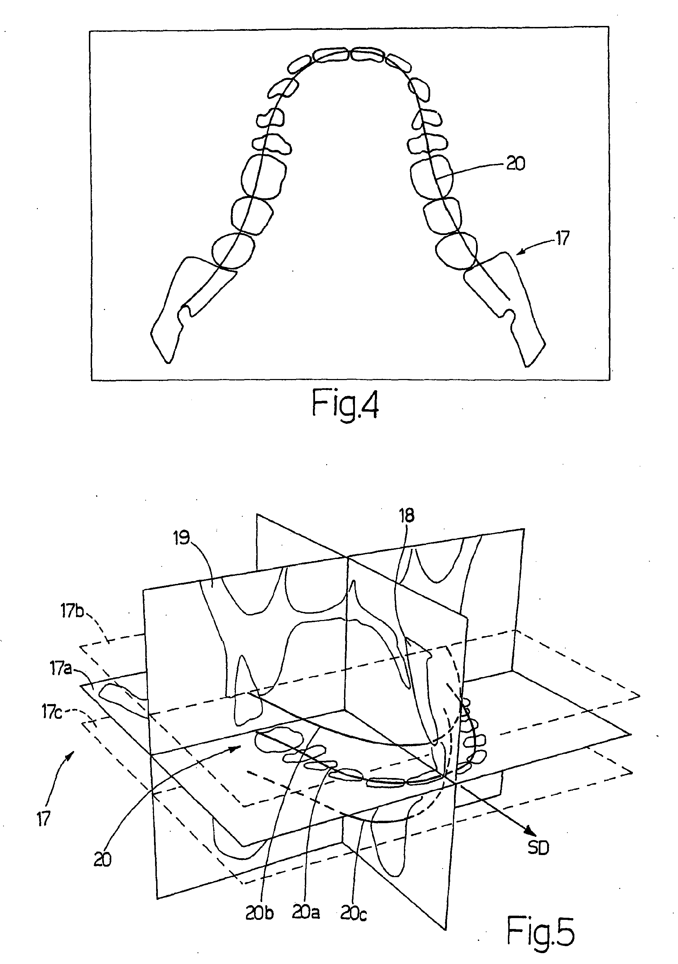

Figure 4 is a schematic illustration of a generic one of the axial sections 17 extracted, which corresponds, by way of example, to the image of Figure 2.

On each axial section 17 extracted, a respective trajectory 20 is determined (Figure 4), which follows substantially a profile of the dental arches in an area corresponding to said axial section 17 extracted. In other words, a trajectory is traced, which follows the curved line according to which the dental arch develops in the axial section 17 in question.

Figure 5 is a schematic illustration of the combination of axial, sagittal, and coronal sections corresponding to the combination of images of Figure 3. On said combination of sections a preferred set of axial sections 17 extracted and an example of respective trajectories 20 is illustrated.

In particular, the axial sections 17 extracted illustrated in Figure 5 are represented, for reasons of simplicity and clarity, by the parallel planes that identify them and comprise a first axial section 17a corresponding to the dental-arch bite, a second axial section 17b corresponding to the upper dental arch, and a third axial section 17c corresponding to the lower dental arch. The trajectories 20 comprise a first trajectory 20a determined on the axial section 17a, a second trajectory 20b determined on the axial section 17b, and a third trajectory 20c determined on the axial section 17c. On account of the different average inclination of the teeth between the two, upper and lower, arches, the different trajectories 20a, 20b, and 20c do not overlap in axial view, and in particular the trajectory 20a, which is traced in an area corresponding to the bite, is more advanced, in a sagittal direction SD, with respect to the other two trajectories 20b and 20c.

The determination of the trajectories is made manually by the operator or else is generated automatically via edge-detection algorithms capable of extracting a profile on the basis of the two-dimensional tomographic data of the corresponding axial section 17 extracted. Manual determination of the trajectories 20 occurs according to two modes, both of which envisage, first of all, display on the monitor 14 of the image of the axial section 17 extracted. According to a first mode, the operator traces the trajectory 20 completely as a broken line, or else as a curved line (for example, of the spline, b-spline, or polynomial type) starting from a plurality of points selected by the operator himself on the image of the axial section 17 displayed. Said points are acquired by the tomography scanner 1 via the keyboard 15 or the mouse 16 of the processing unit 13. In the second mode, displayed together with the image of the axial section 17 extracted is a pre-defined trajectory stored in the processing unit 13 or precalculated via the algorithms mentioned above, and the operator adjusts said pre-defined trajectory to obtain, by changing one or more points as desired of the pre-defined trajectory, the desired trajectory 20. Also in this case, the points that are changed are acquired using the keyboard 15 or the mouse 16.

Next, a surface S (not illustrated) is determined, transverse to the axial sections 17 extracted and such as to comprise all the trajectories 20. The surface S is obtained via interpolation of the trajectories 20, for example, via a linear interpolation. The surface S is, therefore, substantially shaped according to the curved line of the dental arches and according to the average inclination of the teeth of the two, upper and lower, arches.

The panoramic image of the dental arches is generated starting from a part of the volumetric tomographic data identified as a function of the surface S so as to respect the average inclination of the teeth of the two, upper and lower, arches.

In particular, a pair of surfaces SA and SB (not illustrated) is determined such as to define a layer of given thickness which encloses the surface S. The thickness of the layer is determined in such a way as to contain the shapes of the teeth in each of the axial sections 17 extracted. This condition is provided by the alternatives described in what follows that can be selected by the operator.

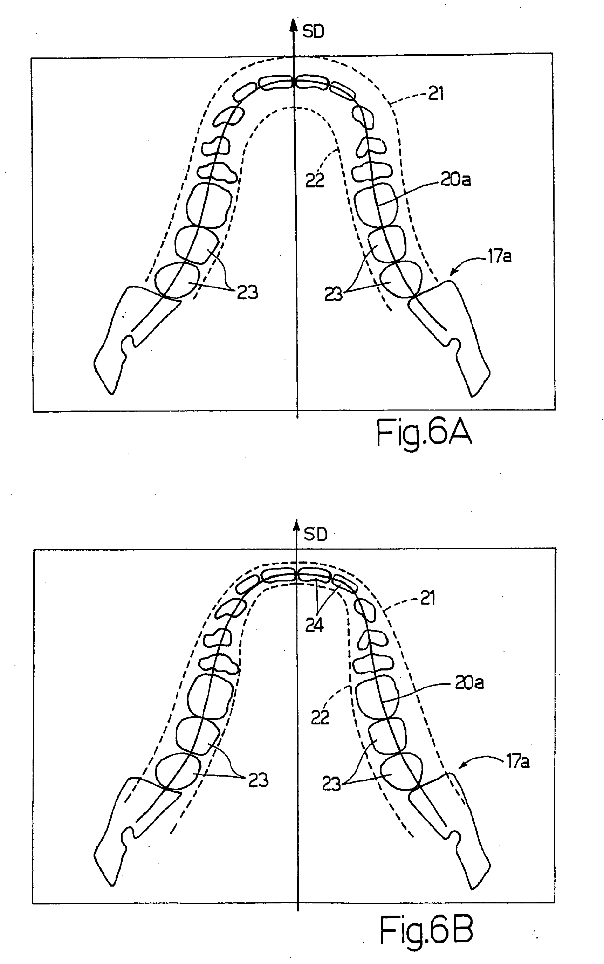

According to a first option, the thickness of the layer is constant along each of the trajectories 20 and is greater than or equal to the maximum of the dimensions of the teeth on the corresponding axial section 17 extracted. Figure 6A illustrates, for example, two lines 21 and 22 defined by the intersection of said layer with the plane that identifies the axial section 17a. As may be noted, the thickness of the layer is substantially equal to the width of the molars 23. According to two variants of said option, the thickness of the layer is constant as the axial sections 17a, 17b, and 17c vary, i.e., it is constant in a direction perpendicular to said sections, or else assumes different values for each of the axial sections 17a, 17b, and 17c. In the first case, the value of the thickness is obviously sized as a function of the maximum of the dimensions of the teeth in all the axial sections 17a, 17b, and 17c.

Alternatively, the thickness of the layer is variable along each of the trajectories 20 so as to follow the variation in the axial dimensions of the teeth along the dental arches. With reference to Figure 6B, the thickness of the layer defined by the lines 21 and 22 is minimum in a region corresponding to the front teeth 24, i.e., along a central portion of the trajectory 20a that intersects the sagittal axis SD, and maximum in a region corresponding to the molars 23. According to two variants of said option, the thickness of the layer remains constant as the axial section 17a, 17b, 17c varies, or else has minimum and maximum values that differ for each axial section.

At this point, the volumetric tomographic data corresponding to the voxels comprised in said layer are combined in a direction orthogonal to said surface S. In particular, for each voxel intercepted by the surface S there is performed an operation of averaging of tomographic data between superimposed voxels in a direction normal to said surface in an area corresponding to the voxel intercepted. The averaging operation is a simple average or else, alternatively, a weighted average. The number of voxels involved in each averaging operation depends upon the thickness of the layer in a region corresponding to the normal directions. The result of the combination is a two-dimensional matrix of values of processed tomographic data, which represents a panoramic image along the surface S.

Finally, the panoramic image is generated by displaying, according to known display techniques, the processed tomographic data of the two-dimensional matrix obtained in the way described above.

The type of thickness of the layer defined by the surfaces SA and SB can be selected by the operator from among the alternatives described previously. In addition, the values of the thickness can be selected by the operator between a minimum value of one voxel and a maximum value of voxels, which can also be set by the operator. For a thickness equal to one voxel, the panoramic image shows substantially a longitudinal section of the teeth. Instead, using a thickness greater than one voxel the image shows a projection of the teeth.

The correspondence of the panoramic image to the actual dimensions and morphology of the dental arches is, obviously, the greater, the higher the number of the axial sections 17 extracted, said number being selectable by the operator. Three sections is in any case the minimum number of axial sections 17 to obtain an advantage over the state of the art, and namely, to adapt the reconstruction of the panoramic image to the substantially different inclination of the axes of the teeth between the upper arch and the lower arch.

According to a further embodiment (not illustrated) of the present invention, which basically constitutes a generalization of the one described above, extracted from the volumetric tomographic data are tomographic data corresponding to at least three sections of the dental arches along respective mutually parallel planes not necessarily orthogonal to the axis 8. This is rendered possible by the fact of having available volumetric tomographic data in digital format and hence easy to process. The further advantage of this embodiment is that it does not require proper positioning a priori of the patient's head. The choice of the orientation of the parallel planes, and hence the point of view of the panoramic image, is determined a posteriori on the data acquired.

From the above description, it may be readily understood that storage of volumetric tomographic data in digital format enables application of the method for the reconstruction of a panoramic image according to the present invention to volumetric tomographic data acquired and reconstructed in any way, and not necessarily according to axial sections. In addition, it is clear that the method for the reconstruction of a panoramic image according to the present invention enables, in the particular case of implementation in the tomography scanner 1 illustrated in Figure 1, panoramic images of any maxillofacial complex to be obtained, or else, in general, panoramic images of any part of the human body or of any object having a not necessarily symmetrical shape.

The main advantage of the method for the reconstruction of a panoramic image according to the present invention is hence that of providing panoramic images of a maxillofacial complex or of any object that respect the actual dimensions of the parts of the object shown in the images. In the particular use in the field of dentistry, the method for reconstruction of a panoramic image according to the present invention and the tomography scanner 1 implementing said method enable adaptation of the reconstruction of the panoramic images of the dental arches to the morphology of the teeth of the individual patient in such a way that said images will represent all the teeth according to their actual dimensions, thus constituting a significant aid to the work of diagnosis of the dentist.

## Claims

1. A method for the reconstruction of a panoramic image of an object; the method comprising:
- acquiring volumetric tomographic data of the object; the method being **characterized in that** it comprises:
- extracting, from the volumetric tomographic data, tomographic data corresponding to at least three sections (17a-17c) of the object identified by respective mutually parallel planes;
- determining, on each section (17a-17c) extracted, a respective trajectory (20a-20c) which substantially follows a profile of the object in an area corresponding to said section (17a-17c);
- determining a first surface (S) transverse to said planes such as to comprise the trajectories (20a-20c); and
- generating the panoramic image on the basis of a part of volumetric tomographic data identified as a function of said surface (S).

2. The method according to Claim 1, in which generating the panoramic image as a function of the data identified by said first surface (S) comprises:
- determining at least two second surfaces (SA, SB) such as to define a layer of given thickness which encloses said first surface (S); and
- combining, in a direction orthogonal to said first surface (S), a part of volumetric tomographic data corresponding to volume elements comprised in said layer.

3. The method according to Claim 2, in which said thickness is variable along each of said trajectories (20a-20c).

4. The method according to Claim 2 or Claim 3, in which said thickness is variable in a direction perpendicular to said parallel planes.

5. The method according to a Claims 2 to 4, in which said combination of said part of volumetric tomographic data envisages carrying out an average between volume superimposed elements in a direction normal to said first surface (S).

6. The method according to any one of Claims 1 to 5, in which each of said trajectories (20a-20c) is generated, via an edge-detection operation, as a function of the tomographic data of the respective said section (17a-17c) of the object.

7. The method according to any one of Claims 1 to 5, in which determination of each of said trajectories (20a-20c) envisages:
- displaying an image of the respective said section (17a-17c) of the object; and
- acquiring trajectory data established by an operator on the basis of said image.

8. The method according to Claim 7, in which the determination of each of said trajectories (20a-20c) envisages:
- modifying a pre-defined trajectory according to said trajectory data established by an operator so as to obtain the desired trajectory (20a-20c).

9. The method according to any one of Claims 1 to 8, in which said first surface (S) is obtained by interpolation of said trajectories (20a-20c).

10. The method according to any one of Claims 1 to 9, in which said object comprises a maxillofacial complex.

11. The method according to Claim 10, in which said maxillofacial complex comprises the dental arches of a patient.

12. The method according to Claim 11, in which said sections (17a-17c) comprise a first section (17a) corresponding to the dental-arch bite, at least one second section (17b) corresponding to the upper dental arch, and at least one third section (17c) corresponding to the lower dental arch.

13. The method according to any one of Claims 2 to 9, in which said object comprises the dental arches of a patient; said sections (17a-17c) comprising a first section (17a) corresponding to the dental-arch bite, at least one second section (17b) corresponding to the upper dental arch, and at least one third section (17c) corresponding to the lower dental arch; said thickness being determined in such a way as to contain the shapes of the teeth of the dental arches in each of the sections (17a-17c).

14. A computer program product that can be loaded into the memory of a processing unit (13) of a computed tomography scanner (1) and is designed for implementing, when run on said processing unit (13), the method according to any one of Claims 1 to 13.

15. A computed tomography scanner comprising: an X-ray source-detector assembly (4), designed to rotate about an area of analysis (5) for acquiring volumetric tomographic data of an object set in the area of analysis (5) itself; processing means (13) for processing the volumetric tomographic data acquired so as to obtain images of the object; display means (14) for displaying the images; and interface means (15, 16) for acquiring data supplied and/or commands imparted by an operator; and being **characterized in that** said processing means (13) are configured for implementing the method according to any one of Claims 1 to 13.

16. A computed tomography scanner for use in dentistry, **characterized in that** it is made according to Claim 15.
